# EUROPEAN PATENT APPLICATION

(11) **EP 1 559 403 A1**
(43) Date of publication of application: **03.08.2005**
(21) Application number: 04001799.8
(22) Date of filing: 28.01.2004
(51) Int. Cl.: A61K 7/13

(54) **Composition for dyeing human hair**

(71) Applicant: KPSS-Kao Professional Salon Services GmbH, 64297 Darmstadt (DE)
(72) Inventor: Wood, Jonathan, Dr., 69469 Weinheim (DE); Wilz, Rüdiger, 64319 Pfungstadt (DE); Nöcker, Bernd, Dr., 64372 Ober-Ramstadt (DE); Kufner, Frank, 64297 Darmstadt (DE); Ghiasi, Fariba, 60316 Frankfurt (DE); Blumenschein, Katrin, 64753 Brombachtal-Hemsbach (DE); Harrèus, Julia, 69469 Weinheim (DE)

(57) **Abstract**

This invention relates to a hair dyeing composition comprising direct acting hair dyes and showing excellent dyeing effects. It should be well understood that the colouring composition of this invention are ready to use colouring composition and, therefore, do not require any mixing prior to application with additional agents such as oxidizers. In another way of carrying out the invention and in the case that a brightening and lightening, in hair colour, is wished (highlighting effect) the compositions of the present invention can as well be used after mixing with oxidizing agent.

## Description

This invention relates to a hair dyeing composition comprising direct acting hair dyes and showing excellent dyeing effects. It should be well understood that the colouring composition of this invention are ready to use colouring composition and, therefore, do not require any mixing prior to application with additional agents such as oxidizers. In another way of carrying out the invention and in the case that a brightening and lightening, in hair colour, is wished (highlighting effect) the compositions of the present invention can as well be used after mixing with oxidizing agent.

Hair colouring is a common practice for ages. Oxidative colouration has widely been used for achieving durable, brilliant hair colour. Direct dyes, mainly of cationic character, have also found their applications for colouring hair. Recently, anionic direct dyes have as well been found to be very powerful for changing hair colour permanently and to achieve long lasting, brilliant colours in strong acidic medium. The colouring agents with anionic dyes are so formulated that the optimum conditions are realised for achieving the highest dyestuff penetration into hair. European patent application with laid open number EP 1 022 014 describes such compositions comprising anionic dyestuffs, solvents, as aid to enhance penetration of said dyestuffs, and a buffer solution to adjust the pH of the dyeing agent in the range from 2 to 6. For enhancing penetration of dyestuffs, solvents are used such as ethanol, benzyl alcohol, propylene carbonate, dipropylene glycol. Products are found on the professional hair dressing market applying this technology.

US 5,601,620, as well, discloses hair colouring agents with acid dyes, an organic solvent and at least one polysiloxane as a conditioner. The dyeing compositions disclosed here are having a pH in the range of 1.5 - 4.5.

Above two documents deal with the anionic dyes in acidic medium and they are silent on any colouring and/or lightening compositions at an alkaline pH and optionally comprising an oxidizing agent.

EP 810 851, on contrary to the above two publications, discloses hair colouring and lightening compositions comprising a cationic direct dye and an oxidizing agent. This documents is totally silent on any composition comprising anionic dyes.

In practice, further need of higher dyeing ability is obviously desired by professional hair dressing practitioners and also end consumers in order to achieve highly brilliant colorations. In addition, partial colourations in the form of streaks has become popular and products are desired showing optimal colouring and at the same time optimal lightening performance.

This invention deals with the above mentioned problems and discloses a hair colouring and lightening composition with excellent colouring and highlighting effects.

Recently, it has surprisingly been found out that hair colouring compositions comprising in aqueous basis direct acting dyes of anionic types, at an alkaline pH show excellent colouring ability and when mixed with an oxidizing agent prior to application as well excellent lightening and colouring performance.

According to the invention the suitable direct acting dyes are acidic, anionic dyes. Those are customarily incorporated in an amount from 0.1 % to 10 %, preferably 0.5 % to 7.5 %, in particular 0.75 % to 7.5 % by weight, calculated to the total composition, into the colouring compositions.

Examples of suitable anionic dyestuffs are:
Acid Black 1, Acid Blue 1, Acid Blue 3, Food Blue 5, Acid Blue 9, Acid Blue 74, Acid Red 14, Acid Red 18, Acid Red 27, Acid Red 52, Acid Red 87, Acid Red 92, Acid Orange 7, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red No. 4, FD&C Yellow No. 6 and Disperse Violet 1 and their alkali metal salts such as sodium, potassium.

Additionally, the colouring compositions according to present invention comprises neutral dyes, so called nitro dyes at a concentration of 0.1 to 7.5%, preferably 0.1 50 5% by weight calculated to total composition.

HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No.15, 2- Amino-6-chloro-4-nitrophenol, picramic Acid, 1,2-Diamino-4-nitrobenzol, 1,4-Diamino-2-nitrobenzol, 3-Nitro-4-aminophenol, 1-Hydroxy-2-amino-3-nitrobenzol and 2-Hydroxyethylpicramic acid.

Direct acting cationic dyes can also be incorporated into the colouring compositions at a concentration less than 0.5%, preferably less than 0.25% by weight calculated to the total composition. It should be kept in mind that those are less suited for the colouring compositions described herein. Examples to those cationic dyes are:
Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Red 2, Basic Red 12 Basic Red 22, Basic Red 76, Basic Violet 1, Basic Violet 2, Basic Violet 3, Basic Violet 10, Basic Violet 14 and Basic Yellow 57.

Plant dyestuffs can also be used alone or in combination with synthetic direct-acting dyestuffs, for example henna (red or black), alkanna root, laccaic acid, indigo, logwood powder, madder root and rhubarb powder, etc.

pH of the coloring compositions of the present invention is alkaline and varies between 8 and 12, preferably 9 - 12, more preferably 9.5 to 11.5. In the case that, the composition is used for highlighting (lightening) and colouring, the forgoing pH values refer to the pH of the dyeing composition before mixing with oxidizing agent. pH of the colouring composition is adjusted to the required pH by using ammonia or its salts with acids such as ammonium chloride, ammonium sulphate, ammonium carbonate, ammonium bicarbonate, ammonium nitrate, or using alkaline solutions such as sodium hydroxide, potassium hydroxide and their respective salts with the known acids.

Colouring composition of present invention can comprise additionally in the base formulation fatty acids with 0 to 3 ethylenic bonds and with fatty acyl chain length of 12 to 22 C atom. Concentration of the fatty acids can be in the range of 0.1 to 10%, preferably 0.1 to 7.5% and most preferably 0.2 to 5% by weight calculated to the total composition. Fatty acid examples, without limiting the choice, suitable for colouring compositions are myristic acid, palmitic acid, behenic acid, steraic acid, oleic acid, linoleic acid. The most preferred fatty acid is oleic acid.

Colouring compositions according to the present invention can be in the form of emulsion, solution, dispersion and/or gel. Emulsion form is preferred.

In the case that the colouring composition is in the form of an emulsion, it comprises as an emulsion base at least one fatty alcohol or mixture of fatty alcohols with the chain length of 14 to 22 C atoms. Examples to suitable fatty alcohols, without limiting the choice, are myristyl alcohol, cetyl alcohol, stearyl alcohol, behenyl alcohol and cetostearyl alcohol. The most preferred is cetostearyl alcohol well known with its trade name Lanette O or as Lanette N in mixture with sodium cetearyl sulfate from Cognis.

The concentration of fatty alcohol(s) is in the range from 0.5 to 20%, preferably 0.5 to 15% by weight, calculated to total composition.

Colouring compositions according to present invention comprises surfactants selected from anionic, nonionic, amphoteric (or zwiterionic) and/or cationic surfactants as emulsifier or solubilizer. Cationic surfactants are as well used as hair conditioners in the colouring composition.

Anionic surfactants suitable within the scope of the invention are in principal known from the cleansing compositions and are preferably present in an amount from 0.1 to about 10%, preferably 0.2 to 7.5% and most preferably 0.2 - 5% by weight, calculated to the total composition.

These are anionic surfactants of the sulfate, sulfonate, carboxylate and alkyl phosphate type, for example, the known C₁₀-C₁₈-alkyl sulfates, and in particular the respective ether sulfates, for example, C₁₂-C₁₄-alkyl ether sulfate, lauryl ether sulfate, especially with 1 to 4 ethylene oxide groups in the molecule, monoglyceride (ether) sulfates, fatty acid amide sulfates obtained by ethoxylation and subsequent sulfatation of fatty acid alkanolamides, and the alkali salts thereof, as well as the salts of long-chain mono- and dialkyl phosphates.

Additional anionic surfactants useful within the scope of the invention are α-olefin sulfonates or the salts thereof, and in particular alkali salts of sulfosuccinic acid semiesters, for example, the disodium salt of monooctyl sulfosuccinate and alkali salts of long-chain monoalkyl ethoxysulfosuccinates.

Suitable surfactants of the carboxylate type are alkyl polyether carboxylic acids and the salts thereof of the formula

R₁ - (C₂H₄O)ₙ - O - CH₂COOX,

wherein R₁ is a C₈-C₂₀-alkyl group, preferably a C₁₂-C₁₄-alkyl group, n is a number from 1 to 20, preferably 2 to 17, and X is H or preferably a cation of the group sodium, potassium, magnesium and ammonium, which can optionally be hydroxyalkyl-substituted, as well as alkyl amido polyether carboxylic acids of the general formula 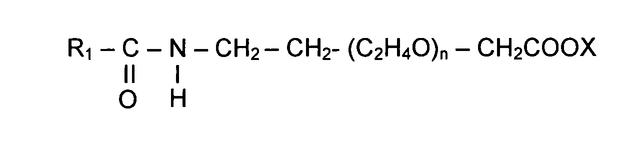 wherein R₁ and X have the above meanings, and n is in particular a number from 1 to 10, preferably 2.5 to 5.

Such products have been known for some time and are on the market, for example, under the trade name "AKYPO®" and "AKYPO-SOFT®".

Also useful are C₈-C₂₀-acyl isethionates, alone or in admixture with other anionic surfactants, as well as sulfofatty acids and the esters thereof.

Further suitable anionic surfactants are also C₈-C₂₂-acyl aminocarboxylic acids or the water-soluble salts thereof. Especially preferred is N-lauroyl glutamate, in particular as sodium salt, as well as, for example, N-lauroyl sarcosinate, N-C₁₂-C₁₈-acyl asparaginic acid, N-myristoyl sarcosinate, N-oleoyl sarcosinate, N-lauroyl methylalanine, N-lauroyl lysine and N-lauroyl aminopropyl glycine, preferably in form of the water-soluble alkali or ammonium, in particular the sodium salts thereof, preferably in admixture with the above-named anionic surfactants.

It is also possible to use mixtures of several anionic surfactants in a mixture.

An overview of the anionic surfactants suitable for the present invention can furthermore be found in the monography of K. Schrader, "Grundlagen und Rezepturen der Kosmetika", 2^{nd} Ed.(1989, Hüthig Buchverlag), pp. 595-600 and pp. 683 to 691.

Further surfactants in the colouring compositions according to the invention are nonionic surfactants alone or in admixture with anionic surfactants at a weight ratio of 3:1 to 1:3.

These are described as well in Schrader, l.c., on pages 600-601 and pp. 694-695.

Especially suited nonionic surfactants are, for example, long-chain fatty acid mono- and dialkanolamides, such as coco fatty acid mono- or diethanolamide and myristic fatty acid mono or diethanolamide, stearic acid mono or diethanolamide.

Further nonionic surfactants suited are alkyl polyglucosides of the general formula

R₂-O-(R₃O)ₙ-Zₓ,

wherein R₂ is an alkyl group with 8 to 18 carbon atoms, R₃ is an ethylene or propylene group, Z is a saccharide group with 5 to 6 carbon atoms, n is a number from 0 to 10 and x is a number between 1 and 5.

Further additionally useful nonionic surfactants are, for example, the various sorbitan esters, such as polyethylene glycol sorbitan stearic acid ester, fatty acid polyglycol esters or poly-condensates of ethyleneoxide and propyleneoxide, as they are on the market, for example, under the trade name "Pluronics^{R}", as well as fatty alcohol ethoxylates.

Further suitable nonionic surfactants are amineoxides. Such amineoxides are state of the art, for example C₁₂-C₁₈- alkyl dimethyl amineoxides such as lauryl dimethyl amineoxide, C₁₂-C₁₈- alkyl amidopropyl or -ethyl amineoxides, C₁₂-C₁₈ -alkyl di(hydroxyethyl) or (hydroxypropyl) amineoxides, or also amineoxides with ethyleneoxide and/or propyleneoxide groups in the alkyl chain. Such amineoxides are on the market, for example, under the trade names "Ammonyx®", "Aromox®" or "Genaminox®".

Further nonionic surfactants useful in the compositions according to invention are C₁₀-C₂₂-fatty alcohol ethoxylates. Especially suited are C₁₀-C₂₂-fatty alcohol ethers, the alkyl polyglycol ethers known by the generic terms "Laureth", "Myristeth", "Oleth", "Ceteth", "Deceth", "Steareth" and "Ceteareth" according to the CTFA nomenclature, including addition of the number of ethylene oxide molecules, e.g., "Laureth-16":

The average degree of ethoxylation thereby ranges between about 2.5 and about 25, preferably about 10 and about 20.

As further surfactant component, the colouring compositions according to the invention can also contain amphoteric or zwitterionic surfactants, for example in an amount from about 0.5 % to about 5 %, preferably from about 1 % to about 2.5 % by weight, calculated to the total composition.

Useful as such are in particular the various known betaines such as alkyl betaines, fatty acid amidoalkyl betaines and sulfobetaines, for example, lauryl hydroxysulfobetaine; long-chain alkyl amino acids, such as cocoaminoacetate, cocoaminopropionate and sodium cocoamphopropionate and -acetate have also proven suitable.

In detail, it is possible to use betaines of the structures 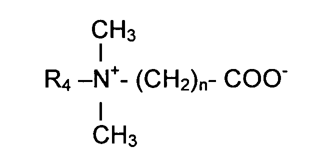 and 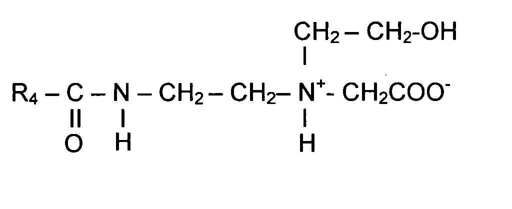 wherein R₄ is a C₈-C₁₈-alkyl group and n is 1 to 3;
sulfobetaines of the structure 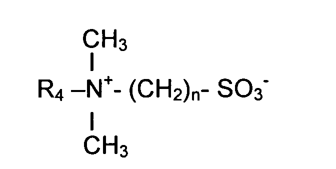 wherein R₄ and n are same as above; and amidoalkyl betaines of the structure 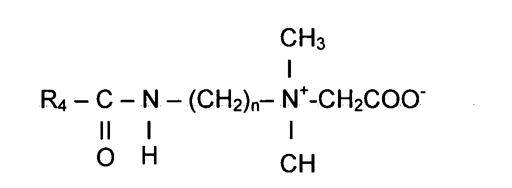 wherein R₄ and n are same as above.

Colouring composition can contain cationic surfactans as emulsifier, solubilizer and/or conditioning ingredients according to the formula, but not limited to. 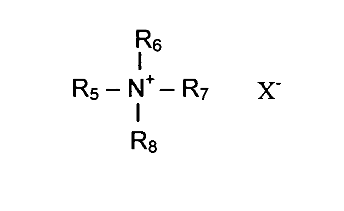 where R₅ is a saturated or unsaturated, branched or non-branched alkyl chain with 8-22 C atoms or

R₉ CO NH (CH₂)ₙ

where R₉ is saturated or unsaturated, branched or non-branched alkyl chain with 7-21 C atoms and n has typical value of 1 - 4 or

R₁₀ CO O (CH₂)ₙ

where R₁₀ is saturated or unsaturated, branched or non-branched alkyl chain with 7-21 C atoms and n has typical value of 1 - 4, and
R₆ is H or unsaturated or saturated, branched or non-branched alkyl chain with 1 - 22 C atoms or

R₉ CO NH (CH₂)ₙ

or

R₁₀ CO O (CH₂)ₙ

where R₉, R₁₀ and n are same as above.

R₇ and R₈ are H or lower alkyl chain with 1 to 4 Carbon atoms, and X is typically chloride, bromide, methosulfate.

Typical examples of those ingredients are cetyl trimethly ammonium chloride, stear trimonium chloride, dipalmitoyl dimonium chloride, distearyl dimethyl ammonium chloride, stearamidopropyl trimonuim chloride, dioleoylethyl dimethyl ammonium methosulfate, dioleoylethyl hydroxyethylmonium methosulfate.

Colouring composition can also contain cationic polymers as conditioning agents. Those are cationic cellulose type polymers know as Polymer JR type from Amerchol such as Polyquaternium 10 or cationic guar gum known with trade name Jaguar from Rhône-Poulenc and chemically for example Guar hydroxypropyl trimonium chloride. Furthermore, chitosan and chitin can also be included in the compositions as cationic natural polymers.

Furthermore, it has been found suitable those cationic polymers known with their CTFA category name Polyquaternium. Typical examples of those Polyquaternium 6, Polyquaternium 7, Polyquaternium 10, Polyquaternium 11, Polyquaternium 16, Polyquaternium 22 and Polyquaternium 28, Polyquaternium 30, Polyquaternium 37, Polyquaternium 36, Polyquaternium 46. Among those the most preferred one is the Polyquaternium 11 as well known with its trade name Gafquat from ISP and as Luviquat PQ from BASF.

As well those polymers known with their CTFA category name Quaternium are suitable. Those are for example Quaternium-8, Quaternium-14, Quaternium-15, Quaternium-18, Quaternium-22, Quaternium-24, Quaternium-26, Quaternium-27, Quaternium-30, Quatemium-33, Quaternium-53, Quaternium-60, Quaternium-61, Quaternium-72, Quaternium-78, Quaternium-80, Quaternium-81, Quaternium-81, Quaternium-82, Quaternium-83 and Quaternium-84.

Typical concentration range for any of the cationic conditioners mentioned above can be 0.01 - 5% by weight, preferably 0.03 - 2.5% by weight and more preferably 0.05 - 1.5% by weight.

Hair dyeing composition of the present invention preferably comprise an organopolysiloxane wherein at least one silicium atom is linked to an alkylene group having a hetero-atom, in particular a nitrogen atom, with a poly-(N-acyl alkyleneimine) with units of the formula 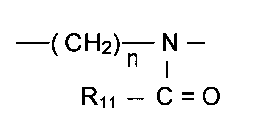 wherein n is a number from 1 to 5 and R₁₁ is hydrogen, a C₁-C₁₂-alkyl or cycloalkyl, aralkyl or aryl group.

Preferred organopolysiloxane polymers are those of the type disclosed in EP-A 640 643, in particular optionally quaternized aminoalkyl, in particular aminopropyl dimethyl polysiloxane/polyethyl oxazoline copolymers of the formula 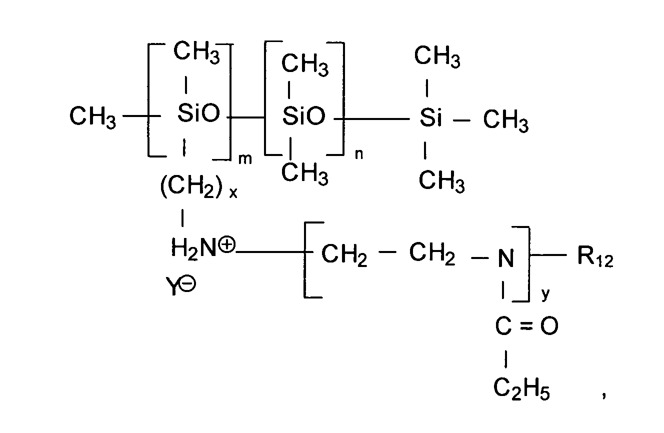 wherein m and n each are numbers from 20 to 10,000, in particular 50 to 7,000, especially 100 to 5,000, x is a number between 1 and 5, preferably 3, and y is a number from 5 to 30, R₁₂ is a C₁-C₁₂-alkyl or aryl group, in particular a methyl, ethyl or benzyl group, and Y⁻ is an anion.

Especially suited are the organopolysiloxanes disclosed under the terms A-1, A-2 and A-3 on pages 12 to 13 of EP-A 640 643. The proportion of graft copolymers in the hair colouring compositions according to the invention ranges from 0.05 % to 5 %, preferably 0.1 % to 2.5 %, in particular 0.5 % to 1.5 % by weight, calculated to the total composition.

Colouring compositions according to the present invention can contain organic solvents as penetration enhancers and also as a solubilzers. Examples of such organic solvents are benzyloxy ethanol, benzyl alcohol, phenoxy ethanol, phenoxy isopropanol, methyl phenoxy ethanol, benzyl glycerol, N-benzyl formide, N-methyl pyrrolidone, N-ethyl pyrrolidone, cinnamyl alcohol, phenethyl alcohol, p-methyl benzyl alcohol, butyl cellosolve, methyl carbitol, ethyl carbitol, propyl carbitol, butyl carbitol, diethyleneglycol, diethyl ether and dipropyleneglycol diethyl ether. Typically the concentration of those solvents can be in the range from 0.5% to 10%, preferably 0.5 - 5% by weight calculated to the total composition.

The hair dyeing compositions according to the invention preferably contain thickening agents. These are, for example, the various cellulose derivatives such as hydroxyalkyl celluloses, e.g. hydroxyethyl cellulose, hydroxypropyl cellulose, hydroxypropyl methyl cellulose, natural polysaccharides such as xanthan gum; guar gum and the alkoxylation products thereof in amounts from 0.1 - 5 %, preferably 0.1 - 3% and most preferably 0.1 - 2% by weight calculated to the total composition and depending on the desired consistency thereof.

Optionally, the colouring composition of this invention can comprise further hair conditioning agents such as silicone oils either volatile or non-volatile, natural and synthetic oils. Among silicone oils those can be added to the colouring composition include dimethicone, dimethiconol, polydimethylsiloxane, DC fluid ranges from Dow Corning, natural oils such as olive oil, almond oil, avocado oil, weizenkeim oil, ricinus oil and the synthetic oils, such as mineral oil, isopropyl myristate, palmitate, stearate and isostearate, oleyl oleate, isocetyl stearate, hexyl laurate, dibutyl adipate, dioctyl - adipate, myristyl myristate and oleyl erucate.

Additional non-ionic conditioning agents may be polyols such as glycerin, glycol and derivatives, polyethyleneglycoles known with trade names Carbowax PEG from Union Carbide and Polyox WSR range from Amerchol, polyglycerin, polyethyleneglycol mono or di fatty acid esters having general formula I or II, respectively,

R₁₃ CO (O CH₂ CH₂)ₙ OH formula I

R₁₃ CO (O CH₂ CH₂)ₙ O OC R₁₄ formula II

where R₁₃ and R₁₄ are independent from each other saturated, unsaturated or branched or non-branched alkyl chain with 7 to 21 C atoms and n is typically 2 - 100.

Another preferred compound in the colouring composition is those of ceramide type according to general formula 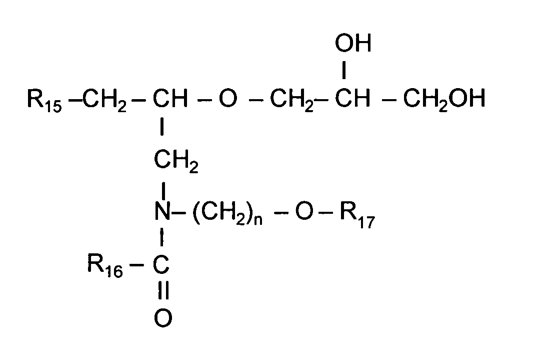 where R₁₅ and R₁₆ are independent from each other alkyl- or. alkenyl group mit 10 to 22 carbon atoms, R₁₆ is methyl, ethyl, n-propyl or isopropyl group and n is a number between 1 to 6, preferably 2 or 3. The concentration of ceramide type of compound in colouring compositions of the present invention can be in the range of 0.01 to 2 and especially 0.01 to 1% by weight calculated to the total composition.

Colouring composition may as well contain UV filters of oil soluble, non-ionic, ones and/or as well those of water soluble and mainly of anionic character. Examples are Benzophenone-1 Benzophenone-2, Benzophenone-3, Benzophenone-7, Benzophenone-6, Benzophenone-8, octylmethoxy cinnamate, homosalat to those of oil soluble ones and Benzophenone-4, benzophenone-9 to those anionic water soluble ones. It should be noted that the other UV filters of oil and water soluble ones should as well be possible to combine.

Another preferred way of carrying out the present invention is that mixing colouring compositions with a composition comprising at least one oxidizing agent prior to application onto hair. By doing so, lightening (highlighting) and colouring is achieved at the same time. In another words, original hair colours is lightened and at the same time dyes, stable in the presence of oxidizing agent, are deposited onto hair so that new colour appearance is achieved. The oxidizing agents suitable are hydrogen peroxide, urea peroxide, melamin peroxide or perborate salts. The most preferred is hydrogen peroxide, which is used as a lotion containing 3 to 12% by weight, calculated to composition only comprising hydrogen peroxide.

The new composition as a result of mixing colouring and oxidizing composition allows achieving simultaneous lightening and colorations. The mixing ratio of the colouring composition and oxidizing composition should be in the range of 4:1 to 1:4, by weight, preferably 2:1 to 1:3 by weight.

Colouring and highlighting with compositions of the present invention can be carried out in different ways of processing.

One of the processes is that colouring composition is mixed with an oxidizing composition and applied onto hair and after a processing time, depending on the wished lightening and as well colour tone, processed for 5 to 45 min and rinsed off from hair.

Another way of carrying out highlighting and colouring is that of two step application. In the first step, composition comprising at least one oxidizing agent is applied onto hair and left on the hair for 5 to 45 min and without rinsing off, the colouring agent is applied onto hair as a second step and after leaving onto hair additional 5 to 45 min the mixed compositions are rinsed of from hair.

In the above mentioned two step colouring and highlighting process, between the application of first and second agents, the hair can be washed with water and optionally hair can be dried.

In the lightening and colouring process using the colouring composition of the present invention, the lightening can as well be carried out with the composition known as bleaching agents. For such a process suitable bleaching composition is for example the one disclosed in a European Patent No 560 088. Preferred way of carrying out lightening an colouring using bleaching agents is two step process as mentioned above.

Another way of carrying out the invention is that addition of oxidation dyestuffs precursors (developing substances) and coupling substances into the colouring compositions of the present invention. Those oxidative dyes can as well be mixed into the colouring composition prior to application onto hair. It is possible to incorporate developing substances known **per se.** In the case of oxidation dyes are present in the compositions, colouring is than carried out in the presence of oxidizing agent, i.e. oxidative dye containing colouring composition is mixed with oxidizing agent prior to application. Special mention is made of p-phenlynediamine, p-methylaminophenol and substituted p-phenylenediamines such as 2,5-diamino-toluene, 2-n-propyl or 2-ethyl-p-phenylenediamine, 2,6-di-methyl-p-phenylene- diamine, 2-(2,5-diaminophenyl) ethanol, 1-amino-4-bis-(2'-hydroxy-ethyl)amino- benzene, 2-(2-hydroxyethyl amino)-5-aminotoluene, 4,4'-diaminodiphenylamine, 4-aminodiphenylamine, 2-amino-5-N,N-diethyl aminotoluene, 4-amino-N-ethyl-N-isopropyl aniline, 2-chloro-p-phenylenediamine, 1-β-hydroxyethyl-2,5-diamino-4-chlorobenzene, 1-β-hydroxyethyl-2,5-diamino-4-methyl benzene, 2-methoxy-p-phenylenediamine, N,N-diethyl-p-phenylenediamine, 1-amino-4-β-methoxyethyl aminobenzene, 1-dimethyl-amino-4-aminobenzene, 1-hydroxy-2,5-diamino-4-methyl benzene, 1-hydroxymethyl-2,5-diaminobenzene, 1,3-dimethyl-2,5-diaminobenzene, 1,4-diamino isopropyl benzene and/or 1-amino-4-β-hydroxypropyl aminobenzene, pyrazole and the derivatives thereof such as 1-hydroxyethyl-4,5-diaminopyrazole, 3,4-diamino-5-hydroxypyrazole, 3,5-diaminopyrazole, 3,5-diamino pyrazol-1-carboxamide, 3-amino-5-hydroxypyrazole, 1-phenyl-2-methylpyrazole, 1-phenyl-3-methylpyrazole-5-one, 3,5-dimethylpyrazole, 3,5-dimethylpyrazole-1-methanol, 3,5-diamino-1,2,4-triazole, 4-aminophenol and the derivatives thereof such as 4-amino-3-methylphenol, 2-chloro-4-aminophenol, 2,6-dichloro-4-aminophenol, 2,4-diamino-phenol, 2,6-dibromo-4-aminophenol, tetraamino pyrimidines, triaminohydroxy pyrimidines, diaminomono- and -dihydroxy pyrimidines, aminotriazines, 5-amino salicylic acid and/or 1,2,4-triamino benzene or the water-soluble salts thereof.

Further suitable aminopyridines are 2,5-diaminopyridine, 2,3-diaminopyridine, 2,6-diaminopyridine, 3-amino-2-methyl amino-6-methoxypyridine, 2-dimethyl-5-aminopyridine, 2-dimethyl aminoethyl-3-hydroxypyridine, 2-amino-4,6-dimethyl pyridine, 2-amino-3-hydroxypyridine, 3-amino-2(β-hydroxyethyl amino)-6-methoxypyridine, 2,6-dimethyl amino-5-aminopyridine, 2-di(hydroxyethyl) amino-5-aminopyridine, 2-hydroxyethyl amino-5-aminopyridine, 4-hydroxy-2,5,6-triaminopyrimidine and/or the water-soluble salts thereof.

The total concentration of the developing substances customarily ranges between about 0.05 % and 5 %, preferably 0.1 % and 4 %, in particular 0.25 % to 0.5 % and 2.5 % to 3 % by weight, calculated to the total hair dyeing composition (excluding the oxidation agent), whereby these figures are always related to the proportion of free base.

The composition according to the present invention can contain coupling substances, which can be selected from 5-amino-2-methylphenol, 2-methyl-5-hydroxyethylaminophenol, 2,4,-diamnophenoxyehanol, 2-amino-4-hydroxyethylaminoanisol, 2-methyl-5-amino-6-chlorphenol, 1,3-bis(2,4-diaminophenoxy)propane, 2-bis(2-hydroxyethyl)aminotoluene, 2-amino-5-methylphenol, resorcinol, 2-methyl resorcinol, 4-chlororesorcinol, 2-amino-4-chlorophenol, 5-amino-4-methoxy-2-methylphenol, 2-aminophenol, 3-amino-phenol, 1-methyl-2-hydroxy-4-aminobenzene, 3-N,N-dimethyl aminophenol, 2,6-dihydroxy-3,5-dimethoxypyridine, 5-amino-3-methylphenol, 6-amino-3-methylphenol, 1 ,3-diamino- benzene, 1-amino-3-(2'-hydroxyethylamino)benzene, 1-amino-3-[bis(2'-hydroxy-ethyl) amino]benzene, α-naphthol, 4,6-dichlororesorcinol, 1,3-diamino-toluene, 4-hydroxy-1,2-methylenedioxy benzene, 1,5-dihydroxy naphthalene, 1,6-dihydroxy naphthalene, 1,7-dihydroxy naphthalene, 2,7-dihydroxy naphthalene, 1-hydroxy-2-methyl naphthalene, 4-hydroxy-1,2-methyldioxy benzene, 2,4-diamino-3-chlorophenol, 5-amino-2-methoxyphenol and/or 1-methoxy-2-amino-4-(2'-hydroxyethyl amino)benzene or the water-soluble salts thereof.

Further, Indole and indoline derivatives can as well be contained in the colouring composition of the present invention. Examples to those are: 6-aminoindol, 6-hydroxyindole, 1-ethyl-6-hydroxyindole, 1-methyl-4-hydroxyindol, 1-methyl-6-hydroxyindole, 2-methyl-6-hydroxyindole, 5-hydroxyindol, 4-hydroxyindol, 5,6-dihydroxyindole, 6-aminoindoline, 6-hydroxyindoline, 1-ethyl-6-hydroxyindoline, 1-methyl-4-hydroxyindoline, 1-methyl-6-hydroxyindoline, 2-methyl-6-hydroxyindoline, 5-hydroxyindoline, 4-hydroxyindoline, 5,6-dihydroxyindoline and their respective salts.

In the hair dyeing compositions according to the invention, the coupling substance(s) as reaction partners of the developing substance(s) are present in approximately the same molecular proportions as the developing substances, i.e. in amounts from 0.01 % to 5.0 %, preferably 0.05 % to 4 %, in particular 0.1 % to 3 % by weight, calculated to the total composition (excluding the oxidizing agent), whereby these figures are always related to the proportion of free base.

The composition of the present invention can contain additional ingredients such as preservatives, chelating agents, fragrance and substances customarily used in cosmetic colouring compositions.

The viscosity of the compositions according to the invention preferably ranges from 1,000 to 50,000, in particular 1,000 to 30,000, especially 1,000 to 20,000 mPa.s, measured at 20°C with a Brookfield rotation viscosimeter, with spindle 5 at 5 rpm.

The invention is illustrated with the following examples, but not limited to.

| **Base formulations** | | |
|---|---|---|
| | % by weight I | % by weight II |
| Stearamide MEA | - | 1.50 |
| Cocamide MEA | 4.00 | 2.00 |
| Cetearyl alcohol | 10.00 | 8.00 |
| Tegin P | 1.40 | 1.40 |
| Propylene Glycol | 2.40 | 1.60 |
| Oleic acid | 3.00 | 3.00 |
| Ammonium chloride | 0.50 | 0.50 |
| Tetrasodium EDTA | 0.20 | 0.20 |
| Sodium lauryl sulfate | 1.50 | - |
| Sodium cetearyl sulfate | - | 1.20 |
| Organopolysiloxane A1 of EP 640 643 | 0.20 | 0.20 |
| Ceramide according to formula wherein | | |
| R15 and R16 are C16 and R17 is ethyl | - | 0.20 |
| | | |
| Water | to 100 | to 100 |

The dyestuffs given in the following as for various colour directions are added to the base compositions either I or II. Water amount is reduced in the base formula corresponding to the amount of dyes present in the formulations.

### Dyestuff composition examples.

| | % by weight | | |
|---|---|---|---|
| | Red-Copper | Copper | Red |
| D&C Yellow 10 | | 1.50 | |
| D&C Oreange 4 | 1.00 | 1.50 | |
| Acid Red 52 | 1.50 | | 1.50 |
| Acid Red 54 | 1.50 | | 1.50 |
| HC Yellow 2 | | 1.50 | |
| 2-Amino-6-chlor-4-nitrophenol | | 3.00 | |
| Base formula | I | I | II |

| | % by weight | | |
|---|---|---|---|
| | Red Violet | Blue | Green |
| D&C Violet 2 | 0.54 | 1.08 | |
| D&C Yellow 10 | | | 1.00 |
| D&C Oreange 4 | | | |
| Acid Black 1 | 0.10 | 0.20 | 0.50 |
| Acid Red 52 | 0.75 | | |
| Acid Red 54 | 0.75 | | |
| Base formula | II | II | 1 |

The colouring compositions so obtained show excellent dyeing performance when applied as it is without premixing with any other composition.

In addition the colouring compositions are mixed with a solution comprising 12% by weight hydrogen peroxide at a ratio of 1:2 (colouring mass : peroxide solution) and applied onto hair, in all cases, excellent highlighting and colouring effects are observed.

## Claims

1. Colouring composition for hair **characterized in that** it comprises at least one anionic direct dye and has a pH from 8 to 12.

2. Colouring composition for hair according to claim 1 **characterized in that** pH of the composition is between 9 and 12.

3. Colouring composition for hair according to claims 1 and 2 **characterized in that** pH of the composition is between 9.5 and 11.5.

4. Colouring composition for hair according to any of the preceding claims **characterized in that** it comprises anionic direct dye at a concentration of 0.1 - 10% by weight, calculated to the total composition.

5. Colouring composition for hair according to any of the preceding claims **characterized in that** anionic direct dye concentration is between 0.5 and 7.5% by weight calculated to the total composition.

6. Colouring composition for hair according to any of the preceding claims **characterized in that** it comprises HC dyes in addition to anionic direct dyes.

7. Colouring composition for hair according to any of the preceding claims **characterized in that** it comprises cationic direct dyes.

8. Colouring composition for hair according to any of the preceding claims **characterized in that** it further comprises saturated and/or unsaturated fatty acids with 0 to 3 ethylenic bonds and a fatty acyl chain length of 12 to 22 C atoms.

9. Colouring composition for hair according to claim 9 **characterized in that** the fatty acid is oleic acid.

10. Colouring composition for hair according to any of the preceding claims **characterized in that** it further comprises fatty alcohol and one or more surfactants selected from anionic, nonionic, amphoteric and/or cationic surfactants.

11. Colouring composition for hair according to claim 11 **characterized in that** anionic surfactants are selected from alkyl sulfates and alkyl ether sulfates, nonionic surfactants are selected from fatty acids mono or diethanolamides and fatty alcohols are with a fatty acyl chain length of 14 to 22 C atoms.

12. Colouring composition for hair according to claims 11 and 12 **characterized in that** it comprises anionic and nonionic surfactants at a weight ratio of 3:1 to 1:3.

13. Colouring composition for hair according to any of the preceding claims **characterized in that** it comprises organosiloxane polymer according to formula 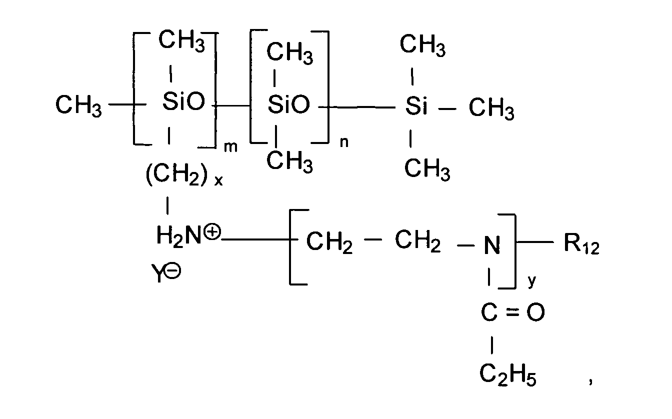 wherein m and n each are numbers from 20 to 10,000, x is a number between 1 and 5, and y is a number from 5 to 30, R₁₂ is a C₁-C₁₂-alkyl or aryl group, in particular a methyl, ethyl or benzyl group, and Y⁻ is an anion.

14. Colouring composition for hair according to any of the preceding claims **characterized in that** it comprises additionally ceramide type compound according to the formula . 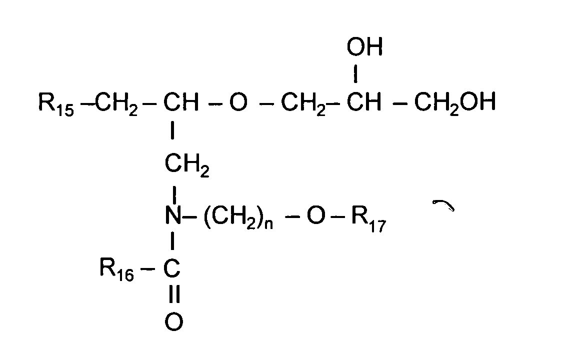 where R₁₅ and R₁₆ are independent from each other alkyl- or. alkenyl group mit 10 to 22 carbon atoms, R₁₇ is methyl, ethyl, n-propyl or isopropyl group and n is a number between 1 to 6, preferably 2 or 3.

15. Colouring composition for hair according to any of the preceding claims **characterized in that** it comprises additionally nonionic thickening polymers selected from cellulose derivatives.

16. Colouring composition for hair according to any of the preceding claims **characterized in that** it comprises additionally organic solvents.

17. Colouring composition for hair according to any of the preceding claims **characterized in that** it comprises additionally UV absorbers selected from anionic water soluble ones and/or nonionic oil soluble ones.

18. Colouring composition for hair according to any of the preceding claims **characterized in that** it is mixed with a composition comprising at least one oxidizing agent prior to application at a weight ratio of 4:1 to 1:4.

19. Colouring composition for hair according claim19 **characterized in that** oxidizing agent is hydrogen peroxide at a concentration of 3 to 12% by weight calculated to total composition.

20. Colouring composition for hair according to claim 19 **characterized in that** it comprises additionally oxidative dyes precursors and coupling substances.

21. Use of colouring composition according to any of the preceding claims for colouring hair.

22. Use of composition according to claims 18 and 19 for colouring and lightening hair.

23. Process for colouring and lightening hair **characterised in that** a composition according to claims 18 and 19 is applied onto hair and left 5 to 45 min and rinsed off from hair.

24. Process for colouring and lightening hair **characterised in that** an oxidizing composition comprising at least one oxidizing agent is applied onto dry hair and left for 5 to 45 min on hair and without rinsing it off from hair a colouring composition according to claims 1-17 is applied onto hair and left further for 5 to 45 min on hair and rinsed off form hair.

25. Process according to claim 24 **characterised in that** before application of colouring composition oxidizing agent is rinsed off from hair and hair dried and subsequently a colouring composition according to claims 1-17 is applied onto hair and left further for 5 to 45 min on hair and rinsed off form hair.
